# EUROPEAN PATENT SPECIFICATION

(11) **EP 2 278 934 B1**
(45) Date of publication and mention of the grant of the patent: **16.09.2015**
(21) Application number: 09734648.0
(22) Date of filing: 23.02.2009
(51) Int. Cl.: A61C 3/02, A61C 8/00, A61B 17/16, A61B 17/58

(54) **BONE REGENERATION DEVICE**
KNOCHENREGENERATIONSVORRICHTUNG
DISPOSITIF POUR LA RÉGÉNÉRATION OSSEUSE

(30) Priority: 25.04.2008 FR 0852799; 29.04.2008 US 48564 P
(43) Date of publication of application: 02.02.2011
(73) Proprietor: Scortecci, Gérard, 06000 Nice (FR)
(72) Inventor: Scortecci, Gérard, 06000 Nice (FR)
(74) Representative: Hautier, Nicolas
(86) International application number: PCT/EP2009/052094
(87) International publication number: WO 2009/130073

(56) References cited:
- WO-A-00/07516
- WO-A-01/97718
- WO-A-02/09598
- WO-A-03/101683
- WO-A-2006/034133
- CN-A- 101 003 889
- JP-A- 2007 181 638

## Description

This invention relates to a device for bone regeneration. It also provides a method for the use of this device such as disclosed in JP2007/81638.

The applications of this invention are manifold, such as devices and therapeutic methods in connection with the maxillomandibular odontostomatology sphere. Implantology, maxillofacial surgery, periodontology, bone necroses, orthodontics, periapical lesions, alveolitis are all part of the scope of the invention. The treatment of other parts of the body, such as, but not limited to the hand, the shoulder, the spinal column or even the knee, is also covered by the possible applications of the invention. Pathologies such as arthrosis and osteoporosis are other possible indications.

The bone tissue is a material in continuous renewal, but its capacity to regenerate varies considerably depending on the sector, as a function of different types of stress: physical (namely applied forces), physiological (muscles, namely blood pressure), pathological (for example, osteoporosis or necrosis) or even traumatic (for example, as a result of an impact or an accident). It constitutes the main metabolic reserve of phosphate and calcium of the human body. The bone tissue is also characterized by a pool of stem cells in the bone marrow, and by cells with a high regenerative potential at its periphery (particularly at the level of the endosteum, the periosteum and stresses the vascular walls). It is the body's framework and it responds to mechanical stresses.

Numerous studies have previously investigated the ability of the bone tissue to repair, regenerate and to remodel itself in various pathologic or traumatic conditions.

More specifically, in order to increase the volume of the bone tissue, we have already proposed the use of multiple and generally complex instruments, for example devices with a bur-like cutting extremity to form cavities in the bone, aiming at a reconstruction at this level. Besides burs, other devices have been proposed, such as drills or other types of osteotomy instruments.

What these different techniques have in common is that they are particularly invasive and they require the surgical raising of a mucoperiosteal flap. Risks of infection, interruption of the bone vascularisation of periosteal origin and post-surgery complications have been observed in many cases.

Other techniques, based on allogenic, autogenous or heterogeneous bone grafts, are also commonly used and provide undeniable services. However, they give rise to new problems, at the level of the recipient site, as well as at the donor site. The overall success rate remains very variable and the complications, not to mention the failures, are far from being insignificant. As mentioned above, these techniques are also particularly complex and invasive.

A need thus exists for a technique that can be used for bone regeneration and is more satisfying than the techniques available up until now.

The present invention falls within the scope of these techniques and provides a solution to all or part of the problems encountered with existing techniques.

According to one aspect of the invention, a device with an end section for penetration through the tissues is proposed.

Applied to the bone tissue, this end section allows the formation of endosseous tracks, able to advantageously reconstruct and modify the initial bone terrain.

For this purpose, the end section has the specific structural characteristics which will be subsequently detailed in the description.

The applicant has established that, owing to its specific properties, the device in question causes, starting with its penetration in the bone tissue, highspeed osteogenic regeneration involving the autologous multi-potent stem cells. In addition to constituting an important technical advance, the surgical act is much less invasive than those required for existing techniques. In fact, the duration of execution and the extent of invasion are reduced. In particular, the extremity of the device easily penetrates through the soft tissues covering the bone tissue and, surprisingly, after the device is pulled out, no notable trace of soft tissue alteration is visible.

Certain effects that may be obtained with the help of this invention are detailed below.

A first effect is the creation of a cortico-trabeculotomy with precipitation of local growth factors and bone morphogenetic proteins (generally abbreviated as BMP) produced by the osteoblasts. These proteins have a fundamental role in bone tissue repair for example in fracture consolidation or bone regeneration. However, these osteo-inducing growth factors are rapidly decomposed by the body. This drawback is overcome by the invention thanks to a release system that makes it possible to potentiate their biologic activity in a localized, controlled, and durable manner.

In the case of this invention, the bone morphogenetic proteins (BMP) induce the recruitment of stem cells from distant sites and the differentiation of mesenchymatous cells into osteoblasts. The device provided by the invention creates an osteogenic bone matrix track with the formation of an intra-osseous and subperiosteal clot. As a result, the initial bone volume is increased.

Besides these potential effects of the invention, the device makes it possible to direct, facilitate, and even re-establish communications and fluid circulation between the periosteum, the endosteum and the bone marrow by creating an intra-matrix mini-tunnel. In addition to these structural changes, the end section of the device causes condensation of the matrix along with variation of the intraosseous pressure. This results in a modification in situ of the compressive stress involving a cascade of cellular mechanoreceptors capable of promoting accelerated bone regeneration, that is quantitatively and qualitatively improved compared to the initial state.

In brief, the device of the invention functions, according to its preferred operating mode, on three simultaneous levels: structural, tensorial and molecular. The bone site is modified favourably. The bone regeneration speed is also noticeably increased compared to existing techniques, for instance due to the fact that molecular transport is facilitated as are self-assemblies, and this also promotes tissue oxygenation and osteogenic remodelling.

Associated with a suitable surgical procedure, the device described herein thus allows the awakening of the cellular activity of the patient's own bone tissue of in healthy, quiescent or pathologic zones by transparietal matricial cortico-trabeculotomy with the device's end section. The spectacular effects produced are partly due to the use of the patient's own reparatory potential, especially his/her own osteogenic stem cells, his/her neoangiogenesis process and the catabolic and anabolic changes that produce a neoformed bone tissue able to resist physiological stresses.

The bone mass is increased by mineralization of the subperiosteal blood clot generated by the passage of the end section of the device.

The device according to this invention may be combined with three-dimensional imaging systems, with computer-generated guides, with aides for navigation, in particular to three-dimensional guidance of the surgical procedure.

Some advantages of the invention that are more specific to the field of odonto-stomatology, are described below. In this field, the bone structure may be degraded in some patients, both qualitatively and quantitatively. The human jawbones consist of two types of anatomically recognizable bone structures, namely the alveolar bone and the denser basal bone, which supports the alveolar bone. The basal bone is the most stable residual structure which is preserved after the loss of teeth. It is worth noting that wearing removable dentures crushes the periosteum, causing ischemia, and gradually resulting in slow, but continuous resorption of the basal bone with the formation of poorly vascularized type I cicatricial bone, particularly in lower jaw.

Type III bone is essentially found in the alveolar bone that is present around the teeth or has not yet completely resorbed, if the teeth have just been extracted. Basal bone can be of type I, II or IV. These are native or cicatricial bone structures, due either to calcium hypercondensation (type I bone) or hypocalcification (type IV bone). Type II bone represents an intermediary stage.

In this context, the invention allows, before a treatment, repair of the recipient bone site both qualitatively and quantitatively as a function of the final therapeutic requirements. The treatments in question include, but are not limited to, orthodontics, implant placement, vertical bone distraction, ridge expansion, sinus lift procedures, transformation of type IV bone into type II bone, revascularization of devascularised type I bone, the acceleration of the reparatory potential of the periosteum and the endosteum, prevention and/or treatment of alveolitis, difficult extractions and non-invasive treatment of periodontal disease.

Other goals and advantages related to the invention will appear further on in the description, which presents a preferred embodiment of the device given as non-limiting example.

Previously, we mentioned that the invention relates to a device for bone regeneration comprising an end section for endo-osseous penetration, characterized in that the end section comprises a primary cylindrical section connected to a conical section ending in a pointed tip. The end section comprises a coating of adamantine carbon with a mirror-polished surface.

A non-exhaustive list of preferred embodiments is given below:
- the primary cylindrical section is connected to the conical section, without discontinuity of diameter,
- the end section includes at least one secondary cylindrical section with a diameter greater than that of the primary cylindrical section,
- the diameter of the cylindrical section or sections is substantially a multiple of 200 micrometers,
- the multiple is chosen as being of 200, 400, 600, 800, 1,000 or 1,200 micrometers,
- the core of the end section is made of stainless steel with a nanotextured surface,
- the Ra roughness of the adamantine carbon coating is less than 0.1 micrometer,
- the device includes an end stop allowing limitation of the penetration of the end section,
- the end stop is adjustable on a micrometrical helical slider,
- the device includes a friction-type gauging ring whose position can be moved relative to the end section,
- the ring is made of elastomer or rubber,
- the end section is mounted on a manually held instrument,
- the end section is mobile in rotation,
- the end section is mobile in percussion by a piezoelectrical drive system,
- the device includes a surgical guide with holes suitable for use with the end section,
- the guide is a clear plastic plate,
- the holes are spaced at least 2 millimetres apart,
- the device includes at least one row of holes in the surgical guide.

The drawings attached herein are given as examples and are not exhaustive for the invention. They only represent an embodiment and allow a better understating of it.
Figure 1 represents a first embodiment of the end section of a device according to the invention.
Figure 2 presents a second embodiment, with two cylindrical sections
Figure 3 shows a section of the anatomic site where the device is to be used
Figure 4 shows the formation of tracks obtained using the end section.
Figures 5a, 5b, 5c, 5d show successive stages in the evolution of the bone tissue during implementation of the method of use of the device.

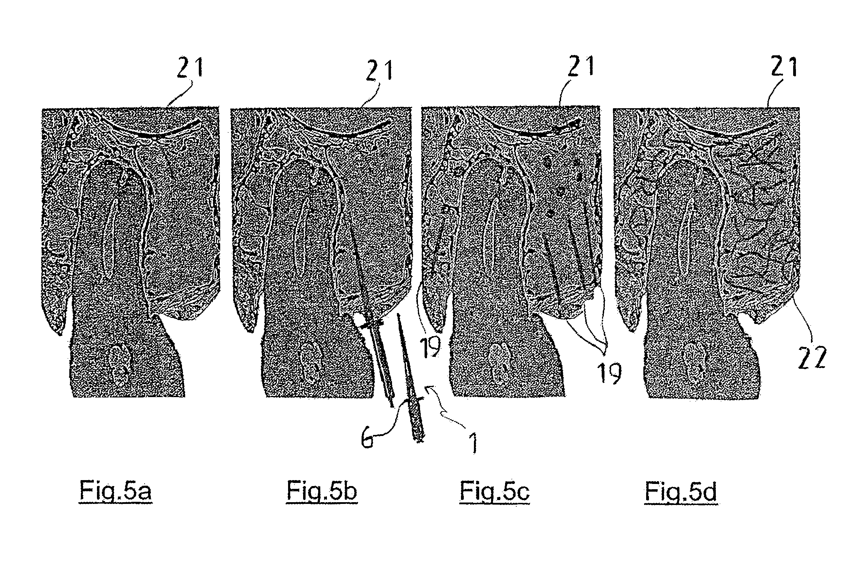

Figure 5a shows the initial bone tissue, figure 5b shows the formation of intra-osseous tracks, figure 5c shows a phase of bone tissue evolution as a result of the formation of tracks, and figure 5d shows the modifications in the bone tissue after a longer period of time.

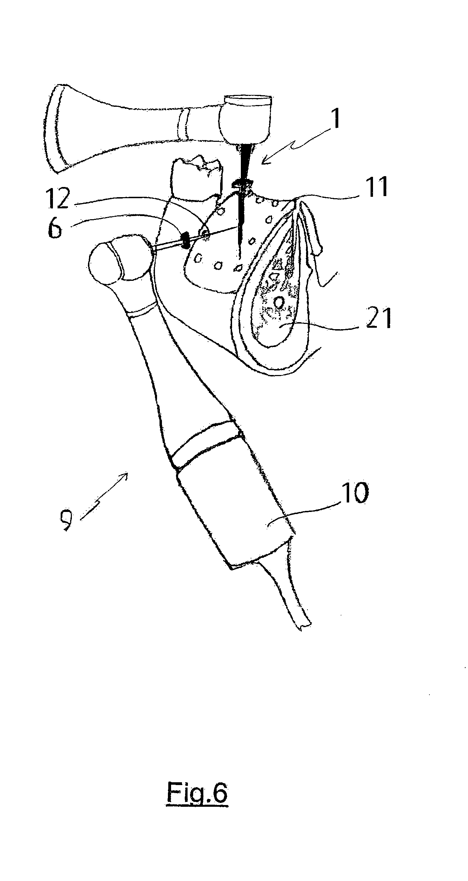

Figure 6 illustrates, in a dental application, the implementation of a device including an end section and an instrument to support it, as well as the formation of several tracks using a surgical guide.

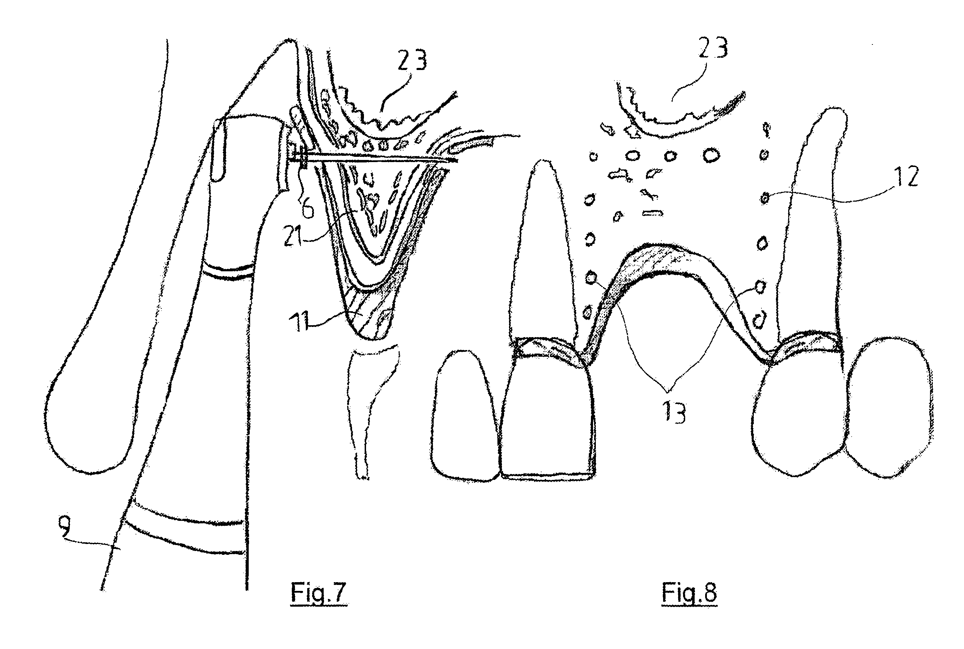

Figures 7 and 8 show, respectively, in cross-section and front view, the formation of transparietal tracks by means of the device in a dental application.

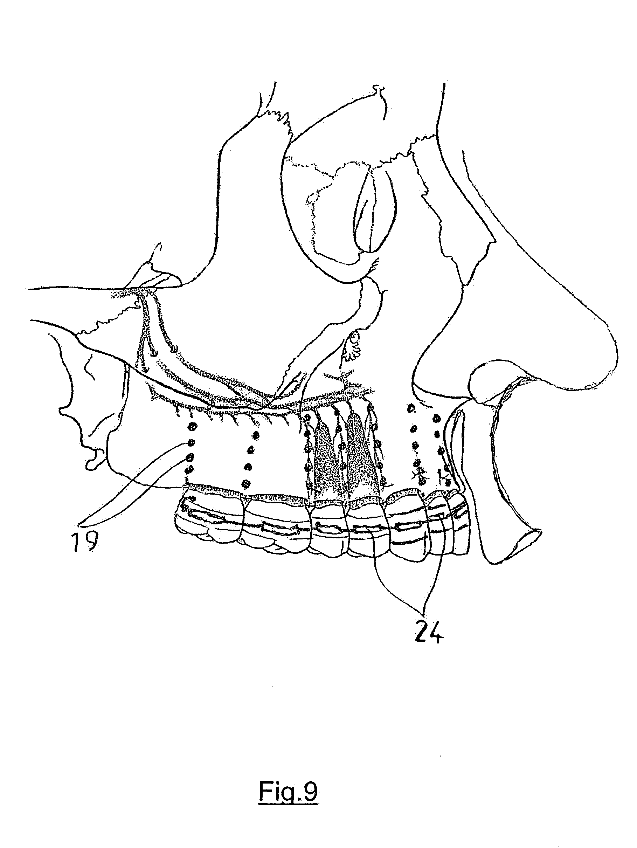

Figure 9 shows the formation of rows of tracks using the invention device, in an orthodontic application.

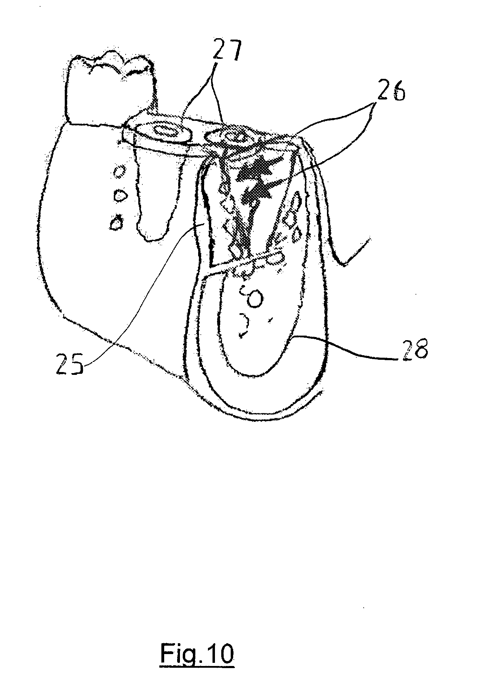

Figure 10 shows, after the stage described in figure 6, horizontal distraction by lower jaw ridge expansion, with placement of implants.

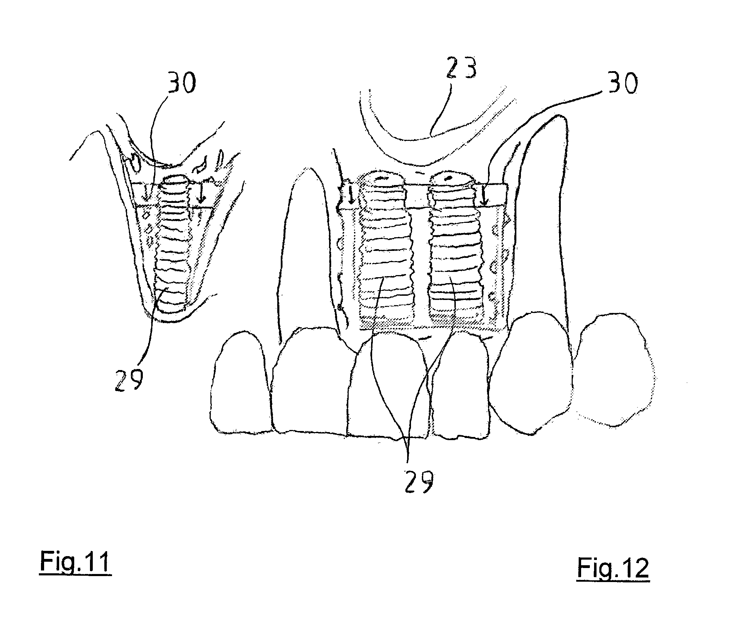

Figures 11 and 12 show an application for vertical distraction with implant placement following the stages shown in figures 7 and 8.

Generally speaking, the method of use of the invention device is as follows: it is a method for bone regeneration consisting in the creation of endosseous tracks and by which:
- the end section 1 is inserted into a predetermined bone sector, penetrating through the adjacent tissues (such as the soft tissues 14);
- the insertion is continued until a predetermined depth is reached;
- the end section 1 is withdrawn immediately, in order to form a track 19 that will be the site of bone reconstruction.

According to preferred variants, given solely for purposes of for information, which are detailed further on in the description:
- several tracks are created in the bone sector, the tracks being advantageously spaced at least 2 mm apart;
- the insertion depth is adjusted by an end stop 6 provided on the end section 1, preferably after analysis of the medical imaging data obtained concerning the bone sector;
- rows of tracks are created in the bone sector;
- the end section 1 is guided by a preformed guide 11 as a function of the bone sector and the external surface of the peripheral tissues;
- the bone reconstruction is gauged, within a predetermined period (21 to 60 days, preferably 45 days), by measuring the depth of insertion of the device in the previously formed track 19.
- the method presented above is used for implant placement, for distraction, or for optimizing an orthodontic treatment.

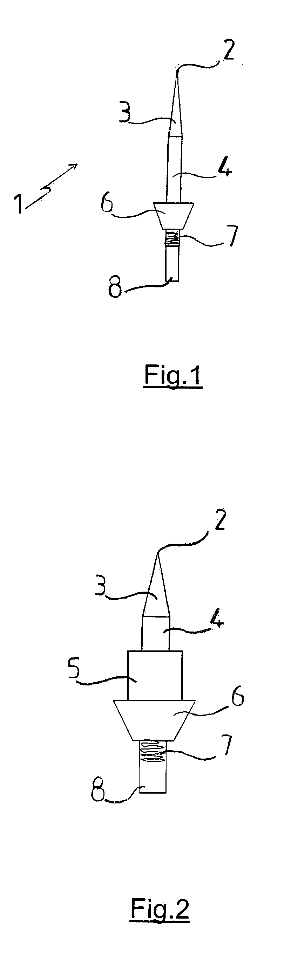

The embodiment described in figure 1 shows an end section 1 able to penetrate human or animal tissues. More exactly, the end section 1 comprises, from its distal section up to its proximal section, a tip 2, a conical section 3, and a cylindrical section 4.

This embodiment also includes an end stop 6, able to limit the penetration of the end section 1 in tissues. Finally, in the proximal zone, a connection area 8 allows mounting, fitting, or any other form of coupling to a support which may be, for instance, a hand held instrument 9, similar to that presented in figures 6 and 7.

The embodiment shown in figure 2 differs from that in figure 1 by the presence of a secondary cylindrical section 5 situated behind the primary cylindrical section 4 and having a greater diameter.

It is mainly preferred that the primary and secondary cylindrical sections 4 and 5 have a circular section.

The portion of the end section 1 that penetrates the tissues is coated with a layer of adamantine carbon (generally abbreviated DLC, for Diamond-Like Carbon) which offers the advantage of great hardness, around 2 500 HV (Vickers hardness), and very low friction coefficient. This coating also presents a particularly smooth, mirror-polished surface, with an arithmetic roughness which may be less than to 0.1 micrometer, for instance 0.06 micrometer. Mirror-polished means that the coating surface is able to reflect light rays due to its smoothness.

A surgical stainless steel, type A440 is used for the core of the end section 1. This type of material is very rigid and has a better fatigue strength than titanium, for example. The combination of this kind of steel with the adamantine carbon coating creates a surface for contact with the tissues that is favourable for the considered application, and a mechanical behaviour which guarantees safe usage, particularly by avoiding the risks of deformation or of rupture of the end section 1. Moreover, the excellent rigidity obtained prevents the end section 1 from bending during penetration, it being understood that such bending could result in inaccuracy during penetration of the tissues and surgical acts that do not comply with the pre-established aims and requirements movements.

Due to the mechanical properties of the materials used, the end section 1 can have a small diameter and the tip 2 is particularly precise, in order to guide the practitioner's movement.

Also preferentially, the primary cylindrical section 4 follows the conical section 3 in its continuity, without any change in diameter. The diameter of the cylindrical sections (primary 4 and possibly secondary 5) is, moreover, chosen in the form of a multiple of the dimension of an osteon, which generally has a circular configuration, with a dimension of about 200 micrometers. For application 5 in the maxillofacial sphere, the diameter of the cylindrical sections 4 and 5 will be chosen as a multiplication coefficient of the osteon, varying between 1 and 6. This diameter is chosen as a function of the therapeutic objective, the concerned region, and the bone density and volume.

In order to promote the bonding of the adamantine carbon coating to the core, the latter is advantageously nanotextured in order to increase its surface and to ensure the three-dimensional character of the bond.

An end section 1 as described herein constitutes a rod that is particularly rigid and specifically selected for minimally traumatic penetration of the different cellular compartments that are crossed successively. It leaves a clean, smooth cylindroconical track in the bone matrix without any contamination of the bone tissue. In the soft tissues, the end section 1 leaves practically no visible trace after it is pulled out. Its passage does not cause any significant trauma, and the tissues heal rapidly by first intention. It does not leave any sequellal and generally does not cause any bleeding in the gum or in the mucosa The advantage of this technique is that the patient being treated does not have to stop taking any blood-twinning drugs he may be on. Treatments with anti-vitamin K agents (AVK) or antiplatelet drugs (such as aspirin) may be continued.

It is important to mention that the adamantine carbon coating is the only surface that comes in contact with the tissues, thus avoiding metallic contaminations and release of cyto-toxic elements. No allergic reaction is possible.

As an example, for a primary cylindrical section 4 with a diameter between 200 and 600 micrometers, a length of about 18mm between the tip 2 and the application surface of the end stop 6, divided into 8 mm for the conical section 3 and 10 mm for the primary cylindrical section 4, has been found satisfactory. The length of the end section 1 susceptible to penetrate in tissues is, moreover, adjustable if the end stop 6 is also adjustable. For this purpose, the end stop 6 is advantageously provided with a threaded inner portion that engages with a corresponding micrometrical thread on a proximal part of the end section 1. Simple rotation of the end stop 6 ensures its perfect adjustment and adjusts the travel of the assembly.

According to a first possibility, the device of the invention is intended for manual use, and it therefore includes a handle element 10 formed on an instrument 9 to which the end section 1 is attached at the connection area 8. In an application in the field of dentistry, a contra-angle handpiece may be used, for example.

However, in other solutions, the end section 1 may be attached to a digitally-controlled support for robotized or remote-controlled operations. In both cases, rotation of the end section 1 is possible via a motorization of standard design. Similarly, the rotation may be replaced or completed by a percussion movement, for example ensured by a piezoelectrical device.

As indicated above, the end section 1 ensures endosseous penetration, particularly for the creation of bone matrix tracks. However, the device according to the invention has a second advantageous mission: to allow, over time, verification of the bone site where the track was created, and namely during the bone reformation. For this purpose, the end section 1 is used by the operator to verify the depth of penetration during the days or the weeks following the creation of the track. In order to gauge this depth, a ring that may be made of natural rubber or elastomer may be configured to move by friction along the end section 1 during its penetration into the previously formed track.

By measuring the distance between the tip 2 and the surface using the ring, the residual depth of the track can be easily determined.

Other gauging devices able to work with the device of the invention are also included in the scope of the invention.

Figures 6 to 8 also present the use of a surgical guide 11 as part of the device of the invention.

In fact, the positioning of the tracks to be created by the end section 1 is advantageously defined by the location of a multiplicity of holes 12 made in a surgical guide 11.

This guide may be namely made of clear plastic (resin) forming a sheet whose inner surface rests on the external wall of the tissues. Preparation of the surgical guide 11 may be carried out after analysis of the radiological data and the aims and requirements established by the practitioner. More specifically, the number of holes 12 and their spacing, as well as their position, are defined in the surgical guide 11. Its general form is, moreover, adapted for accurate positioning on that part of the patient's body to be treated by the device.

The illustration 5 in figures 6 to 8 show the formation of a multiplicity of holes 12 at least some of which are organized in rows 13, whose holes 12 are spaced apart by at least 2 mm. In fact it has been found that this spacing and this organization are satisfactory and yield perfectly conclusive results.

The general method for implementing the device described herein is presented below in more details, with various complements showing examples of application of the invention in the maxillofacial sphere.

The purpose of the invention is, generally speaking, to create a track or a specific non-damaging mini-tunnel that preserves the architecture of the bone matrix and re-establishes the communication of fluids between the periosteum, the matrix, the endosteum, and the bone marrow. It also refers to the generation of a rapid variation of the hydraulic pressure coupled with a modification of the intra-osseous tension by local condensation of the matrix combined with its overall softening by rupture of the type I collagen bundles. In addition, the invention relates to activation of rapid osteogenesis induced by a controlled and configured mini-trauma and the promotion of angiogenesis and oxygenation of the tissues. At the same time, the invention holds the advantage of not causing any adverse reactions such as pain, bleeding, infection, release of cyto-toxins or even cellular transformation and tumour.

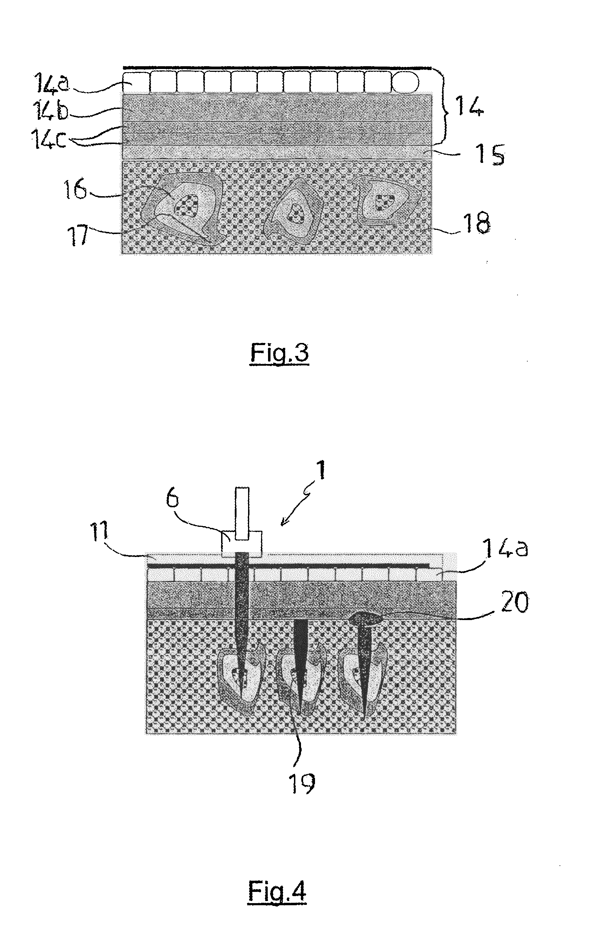

Figure 3 shows the initial state of the bone site that requires intervention. An assembly of referenced soft tissues 14 is comprised of epithelial cells 14a, loose connective tissues 14b, and the periosteum (with two external and internal layers) 14C. The compact bone is represented by reference mark 15 and the bone marrow by reference mark 18. The trabecular bone 16 and the endosteum 17 are also shown with different patterns.

Before the intervention, a surgical guide 11 is fabricated of clear resin. In maxillofacial applications, the guide 11 may be stabilized either by the remaining teeth, or by osteosynthesis screws, or more simply with an oral adhesive.

The fabrication of guide 11 may be performed by traditional laboratory and prosthetic techniques or by computer-assisted means combined with a suitable manufacturing process, such as stereolithography.

In addition to the shape of guide 11, the number of impacts, their direction, their diameter and their depth are determined based on a clinical analysis of the medical imaging data concerning the sector that is to be operated on. A computer-assisted simulation is possible before the surgical act.

In other applications using a transcutaneous approach, namely at the level of the shoulder, hand, knee or vertebrae, the surgical guide 11 may be fixed directly on the skin with a clear suction cup splint system and a navigation system may be used to control the device during its transcutaneous application towards the bone target. The dimensional parameters of the device may be included in a virtual library, allowing a 3D simulation of the future penetration points. Guides 11 are very useful for accurate control of the device; they also serve as means for topological location permitting identification of the impact points made by the end section 1 later on, and especially several weeks after the tracks have been made, in order to validate the effects of the initial passage. The depths of penetration are measured, namely by using a gauging ring, as described above, at the exact place of the initial passage of the end sector 1. This allows verification of the efficacy of the osteogenic response by comparing the initial level and the final level of penetration of the end section 1 when an identical force is applied.

As concerns this subject, figure 3 shows a bone site prior to any use of the device of the invention. Figure 4 shows, from left to right, the penetration of the end section 1 through the soft tissues then into the bone, the creation of the residual track 19 after withdrawal of the device and the evolution of this track in the following minutes. More precisely, as soon as the end section 1 is withdrawn, the track 19 has considerably modified the bone site whereas the soft tissues are not or altered at all or only slightly so. The middle representation of figure 4 illustrates the matrix track two to three seconds after the withdrawal of the end section 1. At this stage, a hemorrhagic phenomenon with bleeding has started. In a later stage, visible on the far right representation of figure 4, the track 19 persists, but is filled in due to the bleeding and the formation of a subperiosteal clot is observed. This is the situation that can be observed around one minute after the device has been withdrawn. Afterwards, and for up to 48 hours after the intervention, the soft tissues 14 resume their initial position, without any macroscopically visible scar: all that occurs is simple cell displacement with instantaneous return to normal. In the soft tissues 14 formed essentially of cells (generally more than 99%), the device thus does not leave any noticeable track during its passage. Repair takes place rapidly, by first intention, this being due to the structure and the material used for the manufacture of the end section 1.

In contrast, the device of the invention aims directly at the bone target which is composed of 99% extra cellular matrix (65% mineral) associated with an organic matrix (35%) containing 90% collagen I.

It is in this solid material forming the bone tissue that the device will leave a long-lasting track. From the first seconds, a haematoma forms in the track 19 left by the device. This is the consequence of rupture of the centromedullary vessels the vascular axes of the interrupted Haversian canals, and the vessels of the periosteum vessels, the endosteum and the bone marrow penetrated by the end section 1. This haematoma remains in situ but it also spreads under the periosteum. It will serve as a frame for the future bone regeneration and it will allow a gain in volume (bone callus).

In the 48 and 72 hours following the intervention, the formation of granulation tissue is initiated. 96 hours after the intervention, there is a small residual clot and the site has been completely filled in by granulation tissue.

At D = 7 days, neoformation of osteoid tissue starts.

At D = 21 days, a peak is observed in osteoclast activity; mineralization of the osteoid tissue by the osteoblasts and neoangiogenesis are seen.

The bone is vitalised and gains flexibility. The cellular mass is considerably increased. The osteoblasts weave the extra-cellular matrix (type I collagen and hydroxypatite). This stage corresponds to that shown in figure 5c. Figure 5a corresponds to the initial osseous tissue which is essentially formed of type IV bone, and figure 5b corresponds to the phase of penetration of the device.

Following the timeline, figure 5d illustrates the situation of the impacted bone site 45 days after the intervention. Woven bone has appeared. Osseous trabeculae are formed and mineralization occurs. The osteoblasts secrete osteoid and type I collagen. A mesh of hydroxypatite is observed in the type I collagen bundles. Preferentially, at this stage, the bone site should be probed using the device, according to the previously presented method by reusing the surgical guide 11 in order to relocate the impact sites. when an identical penetration force is applied, penetration of the end section 1 ceases at a lesser depth than that of the first impact. This reveals the bone regeneration process and allows its quantification.

In the 90 days following the intervention, lamellar type mature bone forms and the initial flexibility is lost. This stiffening is a sign of intensification of the mineralization. At this stage, the bone is sufficiently dense and of good quality for the placement of implants, in odonto-stomatological applications. Once the osteocytes mature, the activity of the osteoblasts ceases. The osteoblasts have transformed into osteocytes which have left the bone tissue to join the pool of border cells at the level of the endosteum, the periosteum, the vascular wall or the bone marrow. In an advantageous manner, a second check of the process's efficacy is carried out at this stage.

The practitioner can then make a decision regarding the continuation of his/her clinical strategy, and namely for the placement of implants.

Generally speaking, the creation of tracks 19 yields the best results when a distance of around 2 mm is left between each impact. This leaves an average of 10 basic bone units (10 osteons) free of any perforation between the tracks 19. This distance is necessary for the maintenance and the cohesion of the local osteo-architecture. Furthermore, as concerns the characteristics of the intervention, it has been noted that a force of 20 N applied by manual pressure is favourable for the creation of tracks 19 whose depth can be an average of 10 mm.

The following text describes some examples of the application of the device of the invention.

One possibility is the use of the device around 45 days prior to implant placement. The device and its application, by the creation of prints and the subsequent process following it (as described above), make it possible to obtain of a well vascularised osseous bed for placement of the selected dental implant which can be a screw-type implant, a disk-form implant, a plate-form implant or any other type in good conditions. In certain cases the implants may be placed earlier, for example around 21 days after the tracks have been created.

As concerns implant placement, the invention may be followed, starting 21 days after the use of the device, by the installation of implant devices of various types. In particular, this could concern the case of axial implants of the type described in publication WO-A1-2006 117298 by the same applicant.

This could also relate to laterally-inserted implants, such as the one presented in publication WO-A1-8504321 by the same applicant.

Another application of the invention is the expansion of narrow ridges (around 2 mm in thickness). The device restores the flexibility of the compact bone, a fact which is interesting in several respects: implant placement in type I bone, horizontal distraction of highly mineralized narrow ridges that are poorly vascularised and contain only minimal cancellous bone and, for which there is a risk of fracture when conventional instruments are applied for this expansion.

In such situations, the invention prepares the bone site for the placement of a distraction device and allows this type of intervention as soon as the bone thickness is at least 2 mm transversally.

It must be noted that, in this application, increases in both volume and bone density can be obtained, without having to make a graft or open the gum. It is estimated that the ridge may increase from 2 mm to 6 mm over the entire expanded height. In this configuration, the device of the invention is applied first of all vertically, using a crestal approach every 2 mm down to a depth of 8 mm along the entire length of the ridge area to be enlarged. After this, the bone sector concerned is penetrated transversally with the end section 1, in parallel manner along the entire height of 10 mm. This operation is shown in figure 6. Ridge expansion is followed by the placement of an expander implant, preferably 21 days after the intervention performed with the device.

Figure 10 shows implant placement 27 and the distraction movement following the direction 26 of the distracted segment 25 relative to the stationary part 28 of the bone.

Figures 7 to 8 show a distraction variant in the form of vertical maxillary distraction before the placement of implants.

Without surgically raising a flap, a U-shaped osteotomy is made using a transmucosal approach.

Advantageously, after 21 days, a distraction device 30 is installed, following by the placement of the implants 29, as shown in figures 11 and 12.

Distraction is performed in only a few minutes, without the need to raise a flap. The implants are left submerged around 6 months.

Another application concerns the field of orthodontics, where transparietal cortico-trabeculotomy can be performed by using the device of the invention.

In this case, and as shown in figure 9, rows 13 of tracks 19 are made after the patient has been fitted with braces using standard orthodontic techniques. A local anaesthesia is given on the vestibular and palatine aspects. The rows of transmucosal cortico-trabeculotomies are, for example, made with an end section 1 mounted on a contra-angle type instrument 9. For this purpose, a guide 11 made of clear resin perforated every 2 mm on both the palatal and lingual surfaces opposite the interradicular spaces is used.

The orthodontic movement is facilitated in this manner, without any radicular resorption. Four to five lateral perforations, extending from one cortical plate to the other on either side of each dental root are sufficient. The cortico-trabeculotomy thus created causes an immediate drop in the intra-osseous pressure by rupture of the type I collagen network. The rigid structure of the bone matrix is softened, allowing the orthodontic movement desired by the practitioner to take place rapidly.

The applications presented above for the invention are not limitative.

**REFERENCES**

| | | | |
|---|---|---|---|
| 1. | End section | | |
| 2. | Tip | | |
| 3. | Conical section | | |
| 4. | Primary cylindrical section | | |
| 5. | Secondary cylindrical section | | |
| 6. | End stop | | |
| 7 | Helical slider | | |
| 8. | Connection area | | |
| 9. | Instrument | | |
| 10. | Handle | | |
| 11. | Surgical guide | | |
| 12. | Holes | | |
| 13. | Row | | |
| 14. | Soft tissues | | |
| 14a. | Epithelial cells | | |
| 14b. | Connective tissues | | |
| 14c. | Periosteum | | |
| 15. | Compact bone | | |
| 16 | Trabecular bone | | |
| 17. | Endosteum | | |
| 18. | Bone marrow | | |
| 19. | Track | | |
| 20. | Clot | | |
| 21. | Bone matrix | | |
| 22. | Type II bone | | |
| 23. | Nasal fossa | | |
| 24. | Orthodontic bracket | | |
| 25. | Distracted segment | 28. | Stationary part |
| 26. | Distraction direction | 29. | Implant |
| 27. | Implant | 30. | Distraction device |

## Claims

1. A device for bone regeneration comprising an end section (1) for endo-osseous penetration,
**characterized in that**
the end section (1) comprises a primary cylindrical section (4) connected to a conical section (3) ending in a tip (2) and the end section (1) has a coating of adamantine carbon with a mirror-polished surface.

2. A device according to claim 1, wherein the primary cylindrical section (4) is connected to the conical section (3), without discontinuity of diameter.

3. A device according to claims 1 or 2, wherein the end section (1) includes at least one secondary cylindrical section (5) with a diameter greater than that of the primary cylindrical section (4).

4. A device according to any one of the preceding claims, wherein the diameter of the cylindrical section or sections (4, 5) is substantially a multiple of 200 micrometers.

5. A device according to claim 4, wherein the multiple is chosen as being of 200, 400, 600, 800, 1,000 or 1,200 micrometers.

6. A device according to any of the preceding claims, wherein the core of the end section (1) is made of stainless steel with a nanotextured surface.

7. A device according to any of the preceding claims, wherein the Ra roughness of the adamantine carbon coating is lesser than 0.1 micrometer.

8. A device according to one of the preceding claims, including an end stop (6) to control the penetration of the end section (1).

9. A device according to claim 8, wherein the end stop (6) is adjustable on a micrometrical helical slider (7).

10. A device according to one of the preceding claims including a friction-type gauging ring that can be moved relative to the end section (1).

11. A device according to claim 10, wherein the ring is made of elastomer or rubber.

12. A device according to any of the preceding claims, wherein the end section (1) is mounted on an instrument (9) for manual handling.

13. A device according to one of the foregoing claims, wherein the end section (1) is mobile in rotation.

14. A device according to one of the foregoing claims, wherein the end section (1) is mobile in percussion by a piezoelectrical drive system.

15. A device according to one of the foregoing claims including a surgical guide (11) with holes suitable for use with the end section (1).

16. A device according to claim 15, wherein the guide (11) is a clear plastic plate.

17. A device according to claim 15 or claim 16, wherein the holes (12) are spaced at least 2 millimetres apart.

18. A device according to one of the claims 15 to 17, including at least one row (13) of holes (12) in the surgical guide (11).

## Patentansprüche

1. Vorrichtung zur Knochenregeneration, umfassend einen Endabschnitt (1) zur endo-ossalen Penetration, **dadurch gekennzeichnet, dass** der Endabschnitt (1) einen primären zylindrischen Abschnitt (4) umfasst, der an einen konischen Abschnitt (3) angeschlossen ist, der in einer Spitze (2) endet, und der Endabschnitt (1) eine Beschichtung aus diamanthartem Karbon mit einer spiegelpolierten Oberfläche hat.

2. Vorrichtung gemäß Anspruch 1, bei der der primäre zylindrische Abschnitt (4) an den konischen Abschnitt (3) ohne Diskontinuität des Durchmessers angeschlossen ist.

3. Vorrichtung gemäß Anspruch 1 oder 2, bei der der Endabschnitt (1) wenigstens einen sekundären zylindrischen Abschnitt (5) mit einem Durchesser einschließt, der größer ist als der primäre zylindrische Abschnitt (4).

4. Vorrichtung gemäß einem der voranstehenden Ansprüche, bei der der Durchmesser des zylindrischen Abschnitts oder der zylindrischen Abschnitte (4, 5) im Wesentlichen ein Vielfaches von 200 Mikrometern ist.

5. Vorrichtung gemäß Anspruch 4, bei der das Vielfache aus 200, 400, 600, 800, 1.000 oder 1.200 Mikrometern ausgewählt ist.

6. Vorrichtung gemäß einem der voranstehenden Ansprüche, bei der der Kern des Endabschnitts (1) aus Edelstahl mit einer nanotexturierten Oberfläche besteht.

7. Vorrichtung gemäß einem der voranstehenden Ansprüche, bei der die Ra-Rauheit der diamantharten Karbonbeschichtung geringer ist als 0,1 Mikrometer.

8. Vorrichtung gemäß einem der voranstehenden Ansprüche, die einen Endanschlag (6) zur Kontrolle der Penetration des Endabschnitts (1) einschließt.

9. Vorrichtung gemäß Anspruch 8, bei der der Endanschlag (6) auf einem mikrometrischen, schneckenförmigen Schieber (7) einstellbar ist.

10. Vorrichtung gemäß einem der voranstehenden Ansprüche, die einen Lehrring vom Reibungstyp einschließt, der relativ zum Endabschnitt (1) bewegt werden kann.

11. Vorrichtung gemäß Anspruch 10, bei der der Ring aus Elastomer oder Gummi besteht.

12. Vorrichtung gemäß einem der voranstehenden Ansprüche, bei der der Endabschnitt (1) auf einem Instrument (9) zur manuellen Handhabung montiert ist.

13. Vorrichtung gemäß einem der voranstehenden Ansprüche, bei der der Endabschnitt (1) in Rotation mobil ist.

14. Vorrichtung gemäß einem der voranstehenden Ansprüche, bei der der Endabschnitt (1) in Perkussion durch ein piezoelektrisches Antriebssystem mobil ist.

15. Vorrichtung gemäß einem der voranstehenden Ansprüche, das eine chirurgische Führung (11) mit für die Verwendung mit dem Endabschnitt (1) geeigneten Löchern einschließt.

16. Vorrichtung gemäß Anspruch 15, bei der die Führung (11) eine durchsichtige Plastikplatte ist.

17. Vorrichtung gemäß Anspruch 15 oder 16, bei der die Löcher (12) mindestens um 2 mm voneinander beabstandet sind.

18. Vorrichtung gemäß Anspruch 15 bis 17, die wenigstens eine Reihe (13) aus Löchern (12) in der chirurgischen Führung (11) einschließt.

## Revendications

1. Dispositif pour la régénération osseuse qui comprend une section d'extrémité (1) pour la pénétration endo-osseuse, **caractérisé en ce que** la section d'extrémité (1) comprend une section cylindrique primaire (4) reliée à une section conique (3) qui se termine par une pointe (2) et **en ce que** la section d'extrémité (1) est pourvue d'un revêtement en carbone adamantin à surface polie miroir.

2. Dispositif selon la revendication 1, dans lequel la section cylindrique primaire (4) est reliée à la section conique (3), sans discontinuité de diamètre.

3. Dispositif selon les revendications 1 ou 2, dans lequel la section d'extrémité (1) comprend au moins une section cylindrique secondaire (5), qui présente un diamètre supérieur à celui de la section cylindrique primaire (4).

4. Dispositif selon l'une quelconque des revendications précédentes, dans lequel le diamètre de la ou des section(s) cylindrique(s) (4, 5) est sensiblement un multiple de 200 micromètres.

5. Dispositif selon la revendication 4, dans lequel le multiple est choisi pour être 200, 400, 600, 800, 1000 ou 1200 micromètres.

6. Dispositif selon l'une quelconque des revendications précédentes, dans lequel le coeur de la section d'extrémité (1) est en acier inoxydable, et présente une surface nanotexturée.

7. Dispositif selon l'une quelconque des revendications précédentes, dans lequel la dureté Ra du revêtement en carbone adamantin est inférieure à 0,1 micromètre.

8. Dispositif selon l'une des revendications précédentes, comprenant une butée d'arrêt (6) permettant de contrôler la pénétration de la section d'extrémité (1).

9. Dispositif selon la revendication 8, dans lequel la butée d'arrêt (6) est réglable sur une glissière hélicoïdale micrométrique (7).

10. Dispositif selon l'une des revendications précédentes, comprenant un anneau de calibrage du type à friction qui peut être déplacé par rapport à la section d'extrémité (1).

11. Dispositif selon la revendication 10, dans lequel l'anneau est composé d'élastomère ou de caoutchouc.

12. Dispositif selon l'une quelconque des revendications précédentes, dans lequel la section d'extrémité (1) est montée sur un instrument (9) de manipulation manuelle.

13. Dispositif selon l'une des revendications précédentes, dans lequel la section d'extrémité (1) est mobile en rotation.

14. Dispositif selon l'une des revendications précédentes, dans lequel la section d'extrémité (1) est mobile en percussion grâce à un système d'entrainement piézoélectrique.

15. Dispositif selon l'une des revendications précédentes comprenant un guide chirurgical (11) muni de trous qui est adapté pour être utilisé avec la section d'extrémité (1).

16. Dispositif selon la revendication 15, dans lequel le guide (11) est une plaque en plastique transparent.

17. Dispositif selon la revendication 15 ou la revendication 16, dans lequel les trous (12) sont espacés les uns des autres d'au moins 2 millimètres.

18. Dispositif selon l'une des revendications 15 à 17, comprenant au moins une rangée (13) de trous (12) prévus dans le guide chirurgical (11).
